# EUROPEAN PATENT APPLICATION

(11) **EP 4 813 312 A1**
(43) Date of publication of application: **30.09.2026**
(21) Application number: 25191800.9
(22) Date of filing: 25.07.2025
(51) Int. Cl.: A61B 6/40, A61B 6/00, A61B 6/51

(54) **ALTERNATING DUAL-ENERGY ORAL SCANNING METHOD, DEVICE, EQUIPMENT, MEDIUM AND PROGRAM**

(30) Priority: 25.03.2025 CN 202510356191
(71) Applicant: Fussen Imaging Co., Ltd., Shenzhen City Guangdong (CN)
(72) Inventor: LI, Huayong, Shenzhen City (CN); FAN, Yi, Shenzhen City (CN); LI, Xunqing, Shenzhen City (CN)
(74) Representative: Chung, Hoi Kan

(57) **Abstract**

The present disclosure relates to the field of medical imaging technology, and proposes an alternating dual-energy oral scanning method, a device, equipment, a medium and a program. The method includes: generating an energy ray beam toward an object to be scanned according to a preset ray generator; performing conversion control on the voltage of the energy ray beam according to preset time intervals to obtain a high-voltage energy ray beam and a low-voltage energy ray beam; using the high-voltage energy ray beam and the low-voltage energy ray beam to perform alternating interval irradiation according to a preset motion trajectory, and collecting attenuation signal data of the high-voltage energy ray beam and the low-voltage energy ray beam in real time; and performing material decomposition on the attenuation signal data to obtain scanning imaging data of the object to be scanned.

## Description

### TECHNICAL FIELD

The present disclosure relates to the technical field of medical imaging, and in particular, to an alternating dual-energy oral scanning method, a device, equipment, a medium and a program.

### BACKGROUND

Cone-beam Computed Tomography (CBCT) is a medical imaging technology widely used in oral and maxillofacial imaging examinations. CBCT has the advantages of high resolution and low radiation dose, and can provide three-dimensional anatomical information to help doctors make more accurate diagnoses and treatment plans.

However, traditional CBCT systems usually use mixed multi-color energy X-ray beams for scanning. After passing through the scanned object, the low-energy part of this beam is easily absorbed to varying degrees, resulting in low accuracy of the attenuation coefficient obtained by tomographic imaging, which is usually accompanied by heavier beam hardening artifacts or metal artifacts. These artifacts will affect the image quality, and in turn cause certain troubles for doctors' clinical examination and diagnosis.

Dual-energy CT can effectively reduce beam hardening artifacts and improve imaging accuracy by acquiring X-ray information of two different types of energy. At present, the dual-energy CT technology has been widely used in spiral CT equipment, which can realize scanning of two energy rays by instantly converting kilovolt mode with a single x-ray tube. However, there are still some problems in the practical application of the existing dual-energy CT technology in the oral field. For example, when the existing dual-energy CT system quickly converts between two types of energy, it would be impossible for tube current to be converted rapidly, resulting in a large dose difference between the two types of energy with the same tube current, which in turn influences the quality of dual-energy imaging and is usually accompanied by heavier beam hardening artifacts or metal artifacts.

### SUMMARY

**In** response to the above problems, an embodiment of the present disclosure provides an alternating dual-energy oral scanning method, a device, equipment, a medium and a program to solve the technical problems of a large dose difference between the two types of energy, influences on the quality of dual-energy imaging, beam hardening artifacts or metal artifacts.

**In** a first aspect, an embodiment of the present disclosure provides an alternating dual-energy oral scanning method, which includes:
generating an energy ray beam toward an object to be scanned according to a preset ray generator;
performing conversion control on the voltage of the energy ray beam according to preset time intervals to obtain a high-voltage energy ray beam and a low-voltage energy ray beam;
using the high-voltage energy ray beam and the low-voltage energy ray beam to perform alternating interval irradiation according to a preset motion trajectory, and collecting attenuation signal data of the high-voltage energy ray beam and the low-voltage energy ray beam in real time; and
performing material decomposition on the attenuation signal data to obtain scanning imaging data of the object to be scanned.

According to an embodiment of the present disclosure, performing conversion control on the voltage of the energy ray beam according to preset time intervals to obtain a high-voltage energy ray beam and a low-voltage energy ray beam includes:
calculating high-voltage irradiation time and low-voltage irradiation time according to a preset high voltage value, a preset low voltage value and preset fluctuation compensation constants;
performing output conversion control on the preset ray generator according to the preset high voltage value and the high-voltage irradiation time to obtain a high-voltage energy ray beam; and
performing output conversion control on the preset ray generator according to the preset low voltage value and the low-voltage irradiation time to obtain a low-voltage energy ray beam.

According to an embodiment of the present disclosure, using the high-voltage energy ray beam and the low-voltage energy ray beam to perform alternating interval irradiation according to a preset motion trajectory includes:
calculating ray beam interval time according to a high voltage value of the high-voltage energy ray beam and a low voltage value of the low-voltage energy ray beam; and
performing alternating interval irradiation on the high-voltage energy ray beam and the low-voltage energy ray beam according to a preset motion trajectory , and to the ray beam interval time.

According to an embodiment of the present disclosure, performing material decomposition on the attenuation signal data includes:
converting the attenuation signal data of the high-voltage energy ray beam and the low-voltage energy ray beam into three-dimensional attenuation image data by back-projection operation to obtain high-voltage volume attenuation image data and low-voltage volume attenuation image data;
performing data decomposition on the high-voltage volume attenuation image data and the low-voltage volume attenuation image data according to preset base material water and preset base material bones to obtain decomposition coefficient image data of water and decomposition coefficient image data of bones; and
calculating virtual monoenergetic image data at a preset energy level according to decomposition coefficient images, and taking the virtual monoenergetic image data as scanning imaging data of the object to be scanned.

According to an embodiment of the present disclosure, performing data decomposition on the high-voltage volume attenuation image data and the low-voltage volume attenuation image data according to preset base material water and preset base material bones to obtain decomposition coefficient image data of water and decomposition coefficient image data of bones includes:
decomposing the high-voltage volume attenuation image data into addition of a product of an attenuation coefficient of water under high-voltage conditions and the decomposition coefficient image data of water and a product of an attenuation coefficient of bones under high-voltage conditions and the decomposition coefficient image data of bones;
using the following formula to decompose the high-voltage volume attenuation image data into addition of the product of the attenuation coefficient of water under high-voltage conditions and the decomposition coefficient image data of water and the product of the attenuation coefficient of bones under high-voltage conditions and the decomposition coefficient image data of bones:
   $\left\{\begin{matrix}{μ}_{h} = {μ}_{ah} A + {μ}_{bh} B \\ {μ}_{l} = {μ}_{al} A + {μ}_{bl} B\end{matrix}\right.$
where *µₕ* and *µₗ* represent the high-voltage volume attenuation image data and the low-voltage volume attenuation image data respectively, *µ*ₐₕ and *µ*_{bh} represent the attenuation coefficient of water under high-voltage conditions and the attenuation coefficient of bones under high-voltage conditions respectively, *µ*ₐₗ and *µ*_{bl} represent the attenuation coefficient of water under low-voltage conditions and the attenuation coefficient of bones under low-voltage conditions respectively, and A and B represent the decomposition coefficient image data of water and the decomposition coefficient image data of bones respectively;
decomposing the low-voltage volume attenuation image data into addition of a product of an attenuation coefficient of water under low-voltage conditions and the decomposition coefficient image data of water and a product of an attenuation coefficient of bones under low-voltage conditions and the decomposition coefficient image data of bones;
calculating the decomposition coefficient image data of water and the decomposition coefficient image data of bones respectively according to the attenuation coefficient of water under high-voltage conditions, the attenuation coefficient of bones under high-voltage conditions, the attenuation coefficient of water under low-voltage conditions, the attenuation coefficient of bones under low-voltage conditions, the high-voltage volume attenuation image data and the low-voltage volume attenuation image data; and
using the following formula to calculate the decomposition coefficient image data of water and the decomposition coefficient image data of bones:
   $\left\{\begin{matrix}A = \frac{{μ}_{h} {μ}_{bl} - {μ}_{l} {μ}_{bh}}{{μ}_{ah} {μ}_{bl} - {μ}_{al} {μ}_{bh}} \\ B = \frac{{μ}_{h} {μ}_{al} - {μ}_{l} {μ}_{ah}}{{μ}_{bh} {μ}_{al} - {μ}_{bl} {μ}_{ah}}\end{matrix}\right.$
where A and B represent the decomposition coefficient image data of water and the decomposition coefficient image data of bones respectively, *µₕ* and represent the high-voltage volume attenuation image data and the low-voltage volume attenuation image data respectively, *µ*ₐₕ and *µ*_{bh} represent the attenuation coefficient of water under high-voltage conditions and the attenuation coefficient of bones under high-voltage conditions respectively, *µ*ₐₗ and *µ*_{bl} represent the attenuation coefficient of water under low-voltage conditions and the attenuation coefficient of bones under low-voltage conditions respectively.

According to an embodiment of the present disclosure, calculating virtual monoenergetic image data at a preset energy level according to decomposition coefficient images includes:
measuring an attenuation coefficient of water at a preset energy level and an attenuation coefficient of bones at a preset energy level respectively; and
calculating virtual monoenergetic image data at the preset energy level according to addition of a product of the attenuation coefficient of water at the preset energy level and the decomposition coefficient image data of water and a product of the attenuation coefficient of bones at the preset energy level and the decomposition coefficient image data of bones.

In a second aspect, an embodiment of the present disclosure provides an alternating dual-energy oral scanning device, which includes:
a ray generation module configured to generate an energy ray beam toward an object to be scanned according to a preset ray generator;
a ray conversion module configured to perform conversion control on the voltage of the energy ray beam according to preset time intervals to obtain a high-voltage energy ray beam and a low-voltage energy ray beam;
an irradiation collection module configured to use the high-voltage energy ray beam and the low-voltage energy ray beam to perform alternating interval irradiation according to a preset motion trajectory, and collecting attenuation signal data of the high-voltage energy ray beam and the low-voltage energy ray beam in real time; and
a data decomposition module configured to perform material decomposition on the attenuation signal data to obtain scanning imaging data of the object to be scanned.

In a third aspect, an embodiment of the present disclosure provides computer equipment, which includes a memory, a processor, and a computer program that is stored in the memory, where the processor executes the computer program to implement the steps of the alternating dual-energy oral scanning method described in the above aspect.

In a fourth aspect, an embodiment of the present disclosure provides a computer-readable storage medium, storing a computer program thereon, where when being executed by a processor, the computer program implements the steps of the alternating dual-energy oral scanning method described in the above aspect.

In a fifth aspect, an embodiment of the present disclosure provides a computer program product, which includes a computer program, where when being executed by a processor, the computer program implements the steps of the alternating dual-energy oral scanning method.

Compared with the existing technologies, the above technical solutions of the present disclosure have the following beneficial effects:
Generating the energy ray beam by the preset ray generator can improve the imaging accuracy and resolution, and can clearly distinguish different tissue structures and lesion areas in the oral cavity; performing conversion control on the voltage of the energy ray beam at preset time intervals to generate the high-voltage and low-voltage energy ray beams can significantly improve imaging accuracy, optimize scanning efficiency, reduce radiation dose, and enhance the material distinguishing ability; using the high-voltage energy ray beam and the low-voltage energy ray beam to perform alternating interval irradiation according to the preset motion trajectory can ensure that after each time of energy conversion, the system has enough time to reach a stable state, ensuring that the collected high and low energy data is accurate and reliable, thereby improving the accuracy of subsequent material decomposition and the quality of final images; performing material decomposition on the attenuation signal data can effectively eliminate artifacts in traditional X-ray imaging, enhance image contrast, and provide tissue attenuation information at specific energy, thereby improving the accuracy and reliability of oral disease diagnosis; by performing data decomposition on the high-voltage volume attenuation image data and the low-voltage volume attenuation image data, decomposition coefficient image data of water and decomposition coefficient image data of bones reflecting the distribution of oral tissue components can be extracted from the dual-energy CT data, providing more accurate information for subsequent tissue analysis and diagnosis, effectively separating and quantifying contributions of different materials, and improving imaging quality and diagnostic accuracy; by calculating the virtual monoenergetic image data at the preset energy level, the oral virtual monoenergetic image at the specific energy can be synthesized. This image has good contrast and clarity, highlights the attenuation differences of different tissues, helps doctors make more accurate diagnoses, and improves quality of imaging.

### BRIEF DESCRIPTION OF DRAWINGS

In order to more clearly illustrate the embodiments of the present disclosure or the technical solutions in the existing technologies, the accompanying drawings required for use in the embodiments or the description of the existing technologies will be briefly introduced below. Obviously, the accompanying drawings described below are only some embodiments of the present disclosure. For those of ordinary skill in the art, other drawings can be obtained based on these drawings without paying creative work.
Fig. 1 shows a flow chart of an alternating dual-energy oral scanning method according to embodiment 1 of the present disclosure;
Fig. 2 shows a functional module diagram of an alternating dual-energy oral scanning device according to embodiment 2 of the present disclosure; and
Fig. 3 shows a schematic diagram of a composition structure of electronic equipment for implementing the alternating dual-energy oral scanning method according to embodiment 3 of the present disclosure.

### DETAILED DESCRIPTION OF THE EMBODIMENTS

The present disclosure is further described below in conjunction with the embodiments shown in the accompanying drawings.

It should be noted that, in the absence of conflict, the embodiments and features in the embodiments of the present application may be combined with each other. The present disclosure will be described in detail below with reference to the accompanying drawings and in conjunction with embodiments.

### Embodiment 1

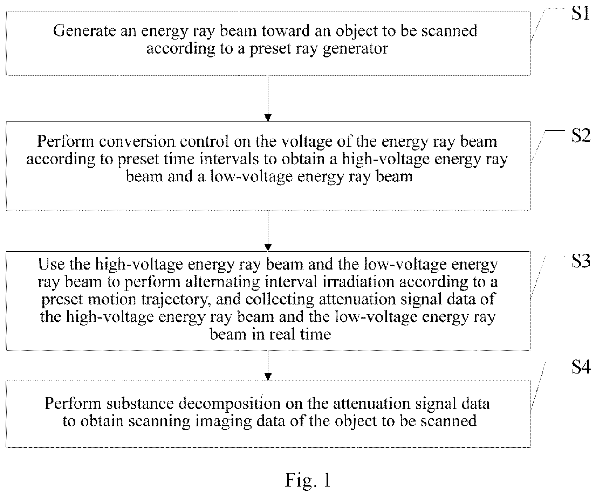

As shown in Fig. 1, an alternating dual-energy oral scanning method provided by an embodiment of the present disclosure includes the following steps:
S1: generating an energy ray beam toward an object to be scanned according to a preset ray generator.

In an embodiment of the present disclosure, the ray generator adopts an X-ray tube, which functions to generate an energy ray beam (X-ray) to penetrate oral tissues to be scanned, and obtains the structural information inside the oral cavity by detecting an attenuation signal of the ray. The core principle of dual-energy scanning is to use the different attenuation characteristics of ray beams of different types of energy (such as high voltage and low voltage) on materials, thereby achieving the distinction and imaging of different materials (such as water and bones).

Further, in dual-energy scanning, the ray generator generates ray beams of different types of energy by adjusting the voltage. High-voltage ray beams have higher penetrating power and are suitable for detecting high-density tissues, while low-voltage ray beams are more suitable for detecting low-density tissues. By alternately generating the high-voltage and low-voltage ray beams and combining them with the processing of attenuation signals, accurate imaging of the internal structure of the oral cavity can be achieved. By generating the energy ray beam toward the object to be scanned, tomographic CT scanning of a skull including the oral cavity can be performed.

In detail, in an embodiment of the present disclosure, step S1 can be implemented by a procedure of initializing the ray generator, setting initial parameters, aiming the X-ray tube at the object to be scanned, starting X-ray tube irradiation, and the detector receiving the attenuation signal after the X-ray beam passes through the scanned object.

In an embodiment of the present disclosure, generating the energy ray beam by the preset ray generator can improve the imaging accuracy and resolution, and can clearly distinguish different tissue structures and lesion areas in the oral cavity.

S2: performing conversion control on the voltage of the energy ray beam according to preset time intervals to obtain a high-voltage energy ray beam and a low-voltage energy ray beam.

In an embodiment of the present disclosure, alternating dual-energy scanning is adopted, and the voltage of the X-ray tube is quickly switched between high voltage (generating high-energy X-rays) and low voltage (generating low-energy X-rays) at preset time intervals through a control circuit. For example, a high voltage of 120 kV is set for irradiation of 5 milliseconds, and then the voltage is switched to a low voltage of 60 kV for irradiation of 5 milliseconds. The cycle is repeated to generate alternating high and low energy ray beams. This alternating dual-energy scanning can effectively utilize the differences in the absorption of high and low energy X-rays in different tissues to achieve material decomposition, improve image quality, reduce artifacts, optimize radiation dose, and reduce equipment cost and complexity compared to dual X-ray tube designs.

In an embodiment of the present disclosure, performing conversion control on the voltage of the energy ray beam according to preset time intervals to obtain a high-voltage energy ray beam and a low-voltage energy ray beam includes:
calculating high-voltage irradiation time and low-voltage irradiation time according to a preset high voltage value, a preset low voltage value and preset fluctuation compensation constants;
performing output conversion control on the preset ray generator according to the preset high voltage value and the high-voltage irradiation time to obtain a high-voltage energy ray beam; and
performing output conversion control on the preset ray generator according to the preset low voltage value and the low-voltage irradiation time to obtain a low-voltage energy ray beam.

In an embodiment of the present disclosure, the fluctuation compensation constants (voltage rise compensation constant C_up and voltage drop compensation constant C_down) are time parameters for correcting the delay and fluctuation of the X-ray tube voltage during the switching process, and are respectively used to make adjustments based on the high-voltage and low-voltage irradiation time to ensure that the actual X-ray energy and intensity generated are as consistent as possible with the preset target values, thereby improving the material decomposition accuracy and image quality of the alternating dual-energy oral scanning.

In an embodiment of the present disclosure, the high-voltage irradiation time and the low-voltage irradiation time can be calculated using the following formula:
${t}_{l} = \frac{{U}_{l}^{2} {αt}_{h}}{{U}_{h}^{2}}$
where U₁ and Uₕ represent voltage values of low energy and high energy settings respectively, and α represents the fluctuation compensation constant, which is set to 1 in this embodiment.

In detail, based on the preset high and low voltage values and the fluctuation compensation constant, the compensated high-voltage and low-voltage irradiation time is calculated respectively; then, the calculated high voltage value and high-voltage irradiation time are used to control the ray generator to output a high voltage energy ray beam; similarly, the low voltage value and the low-voltage irradiation time are used to control the ray generator to output a low-voltage energy ray beam, thereby achieving the alternating emission of high and low energy ray beams.

In an embodiment of the present disclosure, the voltage of the energy ray beam is subjected to conversion control at preset time intervals to generate high-voltage and low-voltage energy ray beams, which can significantly improve imaging accuracy, optimize scanning efficiency, reduce radiation dose, and enhance material distinguishing ability.

S3: using the high-voltage energy ray beam and the low-voltage energy ray beam to perform alternating interval irradiation according to a preset motion trajectory, and collecting attenuation signal data of the high-voltage energy ray beam and the low-voltage energy ray beam in real time.

In an embodiment of the present disclosure, the high-energy and low-energy X-ray beams perform rotational scanning around a patient's oral cavity according to a preset motion trajectory, and the object to be scanned is subjected to alternating interval irradiation. After each time of high-energy or low-energy ray beam irradiation, a detector placed on the opposite side of the oral cavity collects the attenuation signal data of the X-ray after the X-ray penetrates the oral tissue in real time. In this way, the attenuation information of oral tissue under high and low energy can be obtained, providing basic data for subsequent material decomposition. At the same time, the rotational scanning can cover the entire oral cavity, achieve three-dimensional imaging, and ensure that the scanning data has sufficient integrity, thereby improving the scanning coverage and the accuracy of material decomposition.

In an embodiment of the present disclosure, using the high-voltage energy ray beam and the low-voltage energy ray beam to perform alternating interval irradiation according to a preset motion trajectory includes:
calculating ray beam interval time according to a high voltage value of the high-voltage energy ray beam and a low voltage value of the low-voltage energy ray beam;
performing alternating interval irradiation on the high-voltage energy ray beam and the low-voltage energy ray beam according to a preset motion trajectory according to the ray beam interval time.

In an embodiment of the present disclosure, the ray beam interval time can be calculated using the following formula:
${t}_{i} = max \left(t↑\left({U}_{l}, {U}_{h}\right),t↓\left({U}_{h}, {U}_{l}\right),{t}_{d}\right) ∗ s$
where: *t* ↑ (*Uₗ, Uₕ*) is the maximum settling time for the voltage to switch from *Uₗ* to *Uₕ, t* ↓ (*Uₕ, Uₗ*) is the maximum settling time for the voltage to switch from *Uₕ* to *Uₗ,* t_{d} is the maximum response time of the detector, and s is the safety factor (S > 1).

In detail, according to the high and low voltage values, combined with the switching characteristics of the X-ray tube and the detector response time, the appropriate ray beam interval time is calculated using the formula to ensure stable voltage switching and complete data collection. According to the calculated ray beam interval time, high-voltage and low-voltage energy ray beams are emitted alternately on a preset scanning trajectory; after each emission, an interval is waited and then the ray beam is switched to the ray beam of another energy for irradiation. This cycle is repeated to complete the scan of the entire oral cavity.

In an embodiment of the present disclosure, by utilizing the high-voltage energy ray beam and the low-voltage energy ray beam to perform alternating interval irradiation according to the preset motion trajectory, it can be ensured that after each type of energy switching, the system has sufficient time to reach a stable state, ensuring that the collected high and low energy data is accurate and reliable, thereby improving the accuracy of subsequent material decomposition and the quality of final images.

S4: performing material decomposition on the attenuation signal data to obtain scanning imaging data of the object to be scanned.

In an embodiment of the present disclosure, the high-energy and low-energy X-ray attenuation signal data collected by the detector is converted into three-dimensional attenuation images under high and low energy through back-projection operation. The attenuation images are subjected to material decomposition based on preset base materials (such as water and bones) to obtain decomposition coefficient image data of water and decomposition coefficient image data of bones. Finally, by decomposing the coefficient images, a virtual monoenergetic image at a preset energy level is calculated. The virtual monoenergetic image is the final scanning imaging data, which is used for oral diagnosis and analysis.

In an embodiment of the present disclosure, performing material decomposition on the attenuation signal data includes:
converting the attenuation signal data of the high-voltage energy ray beam and the low-voltage energy ray beam into three-dimensional attenuation image data by back-projection operation to obtain high-voltage volume attenuation image data and low-voltage volume attenuation image data;
performing data decomposition on the high-voltage volume attenuation image data and the low-voltage volume attenuation image data according to preset base material water and preset base material bones to obtain decomposition coefficient image data of water and decomposition coefficient image data of bones; and
calculating virtual monoenergetic image data at a preset energy level according to decomposition coefficient images, and taking the virtual monoenergetic image data as scanning imaging data of the object to be scanned.

In an embodiment of the present disclosure, the back-projection operation is used to reconstruct the two-dimensional attenuation signal data collected by the detector, which corresponds to the high-voltage and low-voltage energy ray beams after penetrating the oral tissue, into three-dimensional attenuation image data under high energy and low energy. This process simulates the inverse process of the X-ray penetration path, superimposes the projection data at different angles, and finally restores the three-dimensional spatial distribution information of the oral tissue structure, thereby realizing the conversion from projection data to three-dimensional images.

In an embodiment of the present disclosure, the high-voltage and low-voltage volume attenuation image data are respectively regarded as linear combinations of preset base material water and base material bones. By solving a group of linear equations, the decomposition coefficient image data of water and the decomposition coefficient image data of bones are calculated, thereby reflecting the content distribution of base material water and base material bones in the oral tissue. Using the obtained water decomposition coefficient image data and bone decomposition coefficient image data, as well as the mass attenuation coefficients of these two base materials at the preset energy level, the attenuation coefficient of each voxel at the preset energy level is calculated by weighted summation, thereby synthesizing a virtual monoenergetic image taken at this energy level. This image reflects the attenuation characteristics of the oral tissue at this specific energy and can be used as the final scanning imaging data for diagnosis.

In detail, the two-dimensional attenuation signals collected after high and low energy X-rays penetrate the oral cavity are converted into corresponding three-dimensional volume attenuation images through the back-projection algorithm, and high-voltage and low-voltage volume attenuation image data is obtained respectively; then, based on the preset base material water and base material bones, these attenuation images are subjected to material decomposition to obtain decomposition coefficient image data of water and decomposition coefficient image data of bones, which reflect the distribution of these two materials in the oral tissue; finally, these decomposition coefficients are used to synthesize virtual monoenergetic image data at the preset energy level, which is used as the final scanning imaging data.

In an embodiment of the present disclosure, by performing material decomposition on the attenuation signal data, artifacts in traditional X-ray imaging can be effectively eliminated, image contrast can be enhanced, and tissue attenuation information at specific energy can be provided, thereby improving the accuracy and reliability of oral disease diagnosis.

In an embodiment of the present disclosure, performing data decomposition on the high-voltage volume attenuation image data and the low-voltage volume attenuation image data according to preset base material water and preset base material bones to obtain decomposition coefficient image data of water and decomposition coefficient image data of bones includes:
decomposing the high-voltage volume attenuation image data into addition of a product of an attenuation coefficient of water under high-voltage conditions and the decomposition coefficient image data of water and a product of an attenuation coefficient of bones under high-voltage conditions and the decomposition coefficient image data of bones;
decomposing the low-voltage volume attenuation image data into addition of a product of an attenuation coefficient of water under low-voltage conditions and the decomposition coefficient image data of water and a product of an attenuation coefficient of bones under low-voltage conditions and the decomposition coefficient image data of bones;
calculating the decomposition coefficient image data of water and the decomposition coefficient image data of bones respectively according to the attenuation coefficient of water under high-voltage conditions, the attenuation coefficient of bones under high-voltage conditions, the attenuation coefficient of water under low-voltage conditions, the attenuation coefficient of bones under low-voltage conditions, the high-voltage volume attenuation image data and the low-voltage volume attenuation image data.

In an embodiment of the present disclosure, the high-voltage volume attenuation image data can be decomposed into addition of the product of the attenuation coefficient of water under high-voltage conditions and the decomposition coefficient image data of water and the product of the attenuation coefficient of bones under high-voltage conditions and the decomposition coefficient image data of bones using the following formula;
decomposing the low-voltage volume attenuation image data into addition of a product of an attenuation coefficient of water under low-voltage conditions and the decomposition coefficient image data of water and a product of an attenuation coefficient of bones under low-voltage conditions and the decomposition coefficient image data of bones:
   $\left\{\begin{matrix}{μ}_{h} = {μ}_{ah} A + {μ}_{bh} B \\ {μ}_{l} = {μ}_{al} A + {μ}_{bl} B\end{matrix}\right.$
where *µₕ* and represent the high-voltage volume attenuation image data and the low-voltage volume attenuation image data respectively, *µ*ₐₕ and *µ*_{bh} represent the attenuation coefficient of water under high-voltage conditions and the attenuation coefficient of bones under high-voltage conditions respectively, *µ*ₐₗ and *µ*_{bl} represent the attenuation coefficient of water under low-voltage conditions and the attenuation coefficient of bones under low-voltage conditions respectively, and A and B represent the decomposition coefficient image data of water and the decomposition coefficient image data of bones respectively;
solving the formula to obtain the formula:
   $\left\{\begin{matrix}A = \frac{{μ}_{h} {μ}_{bl} - {μ}_{l} {μ}_{bh}}{{μ}_{ah} {μ}_{bl} - {μ}_{al} {μ}_{bh}} \\ B = \frac{{μ}_{h} {μ}_{al} - {μ}_{l} {μ}_{ah}}{{μ}_{bh} {μ}_{al} - {μ}_{bl} {μ}_{ah}}\end{matrix}\right.$

Therefore, the decomposition coefficient image data of water and the decomposition coefficient image data of bones can be obtained.

In detail, the high-voltage and low-voltage volume attenuation image data is deconstructed into the form of the sums of the products of the attenuation coefficient of water and the attenuation coefficient of bones and the corresponding decomposition coefficient image data, and a linear equation system describing the relationship between the image and the base material is established; then, the water decomposition coefficient image data and the bone decomposition coefficient image data are calculated by solving the linear equation system using the water attenuation coefficient and the bone attenuation coefficient as well as the high and low voltage volume attenuation image data.

In an embodiment of the present disclosure, by performing data decomposition on the high-voltage volume attenuation image data and the low-voltage volume attenuation image data, water decomposition coefficient image data and bone decomposition coefficient image data reflecting the distribution of oral tissue components can be extracted from the dual-energy CT data, thereby providing more accurate information for subsequent tissue analysis and diagnosis, effectively separating and quantifying the contributions of different materials, and improving imaging quality and diagnostic accuracy.

In an embodiment of the present disclosure, calculating virtual monoenergetic image data at a preset energy level according to decomposition coefficient images includes:
measuring an attenuation coefficient of water at a preset energy level and an attenuation coefficient of bones at a preset energy level respectively; and
calculating virtual monoenergetic image data at the preset energy level according to addition of a product of the attenuation coefficient of water at the preset energy level and the decomposition coefficient image data of water and a product of the attenuation coefficient of bones at the preset energy level and the decomposition coefficient image data of bones.

In an embodiment of the present disclosure, the attenuation coefficient of water at the preset energy level and the attenuation coefficient of bones at the preset energy level refer to the degree to which a material absorbs or scatters X-rays at specific energy, which are important parameters for weighted calculation when synthesizing the virtual monoenergetic image, ensuring that the synthesized virtual monoenergetic image can accurately reflect the attenuation characteristics of oral tissue at this energy.

In an embodiment of the present disclosure, the following formula may be used to calculate virtual monoenergetic image data at the preset energy level by adding the product of the attenuation coefficient of water at the preset energy level and the decomposition coefficient image data of water and the product of the attenuation coefficient of bones at the preset energy level and the decomposition coefficient image data of bones:
${μ}_{E} = {μ}_{a_E} A + {μ}_{b_E} B$
where: A and B represent the decomposition coefficient image data of water and the decomposition coefficient image data of bones, respectively, and *µ*_{a_E} and *µ*_{b_E} represent the attenuation coefficient of water at the preset energy level and the attenuation coefficient of bones at the preset energy level, respectively.

In an embodiment of the present disclosure, by calculating the virtual monoenergetic image data at the preset energy level, the oral virtual monoenergetic image at specific energy can be synthesized. The image has good contrast and clarity, highlights the attenuation differences of different tissues, helps doctors make more accurate diagnoses, and improves quality of imaging.

### Embodiment 2

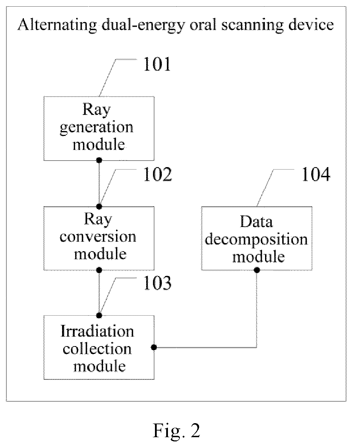

As shown in Fig. 2, this embodiment further provides a functional module diagram of an alternating dual-energy oral scanning device.

The alternating dual-energy oral scanning device 100 described in this embodiment may be installed in electronic equipment. According to implemented functions, the alternating dual-energy oral scanning device 100 may include a ray generation module 101, a ray conversion module 102, an irradiation collection module 103 and a data decomposition module 104. The module described in the present disclosure may also be referred to as a unit, which refers to a series of computer program segments that can be executed by a processor of electronic equipment and can complete fixed functions, and is stored in a memory of the electronic equipment.

In this embodiment, the functions of each module/unit are as follows:
a ray generation module 101 is configured to generate an energy ray beam toward an object to be scanned according to a preset ray generator;
a ray conversion module 102 is configured to perform conversion control on the voltage of the energy ray beam according to preset time intervals to obtain a high-voltage energy ray beam and a low-voltage energy ray beam;
an irradiation collection module 103 is configured to use the high-voltage energy ray beam and the low-voltage energy ray beam to perform alternating interval irradiation according to a preset motion trajectory, and collecting attenuation signal data of the high-voltage energy ray beam and the low-voltage energy ray beam in real time; and
a data decomposition module 104 is configured to perform material decomposition on the attenuation signal data to obtain scanning imaging data of the object to be scanned.

In detail, each module described in the alternating dual-energy oral scanning device 100 described in an embodiment of the present disclosure adopts the same technical means as the alternating dual-energy oral scanning method described in embodiment 1 when in use, and can produce the same technical effects, which will not be repeated here.

### Embodiment 3

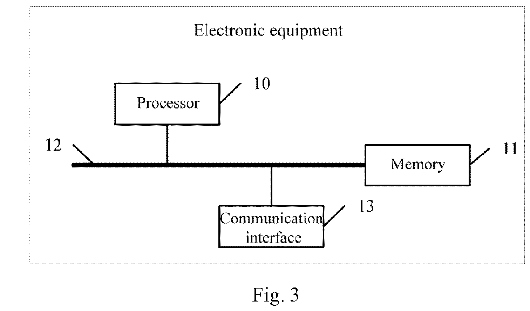

As shown in Fig. 3, this embodiment also provides computer electronic equipment, which may include a processor 10, a memory 11, a communication bus 12, and a communication interface 13, and may also include a computer program stored in the memory 11 and executable on the processor 10, such as an alternating dual-energy oral scanning program.

Specifically, the processor 10 may be composed of an integrated circuit, for example, a single packaged integrated circuit, or a plurality of packaged integrated circuits with the same or different functions, including one or more central processing units (CPUs), microprocessors, digital processing chips, graphics processors, and a combination of various control chips. The processor 10 is the control unit of the electronic equipment, and uses various interfaces and lines to connect the various components of the entire electronic equipment. The processor executes various functions of the electronic equipment and processes data by running or executing programs or modules stored in the memory 11 (for example, executing an alternating dual-energy oral scanning program, etc.) and calling data stored in the memory 11.

The memory 11 includes at least one type of readable storage medium, and the readable storage medium includes a flash memory, a mobile hard disk, a multimedia card, a card-type memory (for example: SD or DX memory, etc.), magnetic memory, magnetic disk, optical disk, etc. In some embodiments, the memory 11 may be an internal storage unit of the electronic equipment, such as a mobile hard disk of the electronic equipment. In other embodiments, the memory 11 may also be external storage equipment of the electronic equipment, such as a plug-in mobile hard disk, a smart media card (SMC), a secure digital (SD) card, a flash card, etc. equipped on the electronic equipment. Further, the memory 11 may include both an internal storage unit of the electronic equipment and external storage equipment. The memory 11 can be used not only to store application software and various data installed in the electronic equipment, such as the code of the alternating dual-energy oral scanning program, but also to temporarily store data that has been output or is to be output.

The communication bus 12 may be a peripheral component interconnect (PCI) bus or an extended industry standard architecture (EISA) bus. The bus can be divided into address bus, data bus, control bus, etc. The bus is configured to implement connection and communication between the memory 11 and at least one processor 10 and the like.

The communication interface 13 is used for communication between the electronic equipment and other devices, and includes a network interface and a user interface. Optionally, the network interface may include a wired interface and/or a wireless interface (such as a WI-FI interface, a Bluetooth interface, etc.), which is generally used to establish a communication connection between the electronic equipment and other electronic equipment. The user interface may be a display or an input unit (such as a keyboard). Optionally, the user interface may also be a standard wired interface or a wireless interface. Optionally, in some embodiments, the display may be an LED display, a liquid crystal display, a touch-sensitive liquid crystal display, an OLED (Organic Light-Emitting Diode) touch device, and the like. The display may also be appropriately referred to as a display screen or a display unit, which is used to display information processed in the electronic equipment and to display a visual user interface.

The figure only shows an electronic equipment with components. Those skilled in the art will understand that the structure shown in the figure does not constitute a limitation on the electronic equipment, and may include fewer or more components than shown in the figure, or combine certain components, or arrange the components differently.

For example, although not shown, the electronic equipment may also include a power source (such as a battery) for supplying power to each component. Preferably, the power source may be logically connected to the at least one processor 10 via a power management device, thereby implementing functions such as charging management, discharging management, and power consumption management through the power management device. The power source may also include one or more DC or AC power sources, recharging devices, power failure detection circuits, power converters or inverters, power status indicators, and other arbitrary components. The electronic equipment may also include a variety of sensors, Bluetooth modules, Wi-Fi modules, etc., which will not be described in detail here.

It should be understood that the embodiment is for illustration only and the scope of the patent application is not limited to this structure.

The alternating dual-energy oral scanning program stored in the memory 11 of the electronic equipment is a combination of multiple instructions, which, when running in the processor 10, can implement:
generating an energy ray beam toward an object to be scanned according to a preset ray generator;
performing conversion control on the voltage of the energy ray beam according to preset time intervals to obtain a high-voltage energy ray beam and a low-voltage energy ray beam;
using the high-voltage energy ray beam and the low-voltage energy ray beam to perform alternating interval irradiation according to a preset motion trajectory, and collecting attenuation signal data of the high-voltage energy ray beam and the low-voltage energy ray beam in real time; and
performing material decomposition on the attenuation signal data to obtain scanning imaging data of the object to be scanned.

Specifically, the specific implementation method of the processor 10 for the above instructions can refer to the description of the relevant steps in the corresponding embodiment of the accompanying drawings, which will not be repeated here.

Further, if the module/unit integrated in the electronic equipment is implemented in the form of a software functional unit and sold or used as an independent product, it can be stored in a computer-readable storage medium. The computer-readable storage medium may be volatile or nonvolatile. For example, the computer-readable medium may include: any entity or device, recording medium, USB flash drive, mobile hard disk, magnetic disk, optical disk, computer memory, read-only memory (ROM) that can carry the computer program code.

### Embodiment 4

This embodiment provides a storage medium, storing a computer program. When being executed by a processor, the computer program implements the steps of the alternating dual-energy oral scanning method described above.

These program codes may also be loaded onto a computer or other programmable data processing equipment, so that a series of operation steps are executed on the computer or other programmable device to produce a computer-implemented process. Hence, the instructions executed on the computer or other programmable equipment provide steps for implementing the functions specified in one or more processes of the flow chart.

Storage media include permanent and non-permanent, removable and non-removable media, and information storage can be achieved by any method or technology. The information may be computer-readable instructions, data structures, program modules or other data. Examples of storage media may include, but are not limited to, phase change memory (PRAM), static random access memory (SRAM), dynamic random access memory (DRAM), other types of random access memory (RAM), read-only memory (ROM), electrically erasable programmable read-only memory (EEPROM), flash memory or other memory technology, compact disk-read-only memory (CD-ROM), digital versatile disk (DVD) or other optical storage, magnetic cassettes, disk storage or other magnetic storage devices, or any other non-transmission medium that can be used to store information that can be accessed by a computing device.

In several embodiments provided by the present disclosure, it should be understood that the disclosed equipment, devices and methods may be implemented in other ways. For example, the device embodiments described above are merely illustrative. For example, the division of the modules is merely a logical function division, and there may be other division methods in actual implementation.

The modules described as separate components may or may not be physically separated, and the components shown as modules may or may not be physical units, that is, they may be located in one place or distributed on multiple network units. Some or all of the modules may be selected according to actual needs to achieve the purpose of the solution in this embodiment.

In addition, each functional module in each embodiment of the present disclosure may be integrated into one processing unit, or each unit may exist physically separately, or two or more units may be integrated into one unit. The above integrated unit can be implemented in the form of hardware or in the form of hardware plus software functional modules.

It is obvious to those skilled in the art that the present disclosure is not limited to the details of the above exemplary embodiments, and that the present disclosure can be implemented in other specific forms without departing from the spirit or essential characteristics of the present disclosure.

Therefore, no matter from which point of view, the embodiments should be regarded as illustrative and non-restrictive, and the scope of the present disclosure is not limited only according to the above description, and it is intended that all changes within the meaning and scope of equivalent elements within the scope of protection are included in the present disclosure.

The embodiments of the present application can acquire and process relevant data based on artificial intelligence technology. , Artificial Intelligence (AI) is the theory, method, technology and application system that uses digital computers or machines controlled by digital computers to simulate, extend and expand human intelligence, perceive the environment, acquire knowledge and use knowledge to obtain the best results.

In addition, it is obvious that the word "include" does not exclude other elements or steps and the singular does not exclude the plural. Multiple units or devices stated in the system claims may also be implemented by one unit or device through software or hardware. The words first, second, etc. are used to indicate names and do not indicate any particular order.

Finally, it should be noted that the above embodiments are only used to illustrate the technical solutions of the present disclosure rather than to limit them. Although the present disclosure has been described in detail with reference to the preferred embodiments, those skilled in the art should understand that the technical solutions of the present disclosure can be modified or replaced by equivalents without departing from the spirit and scope of the technical solution of the present disclosure.

## Claims

1. An alternating dual-energy oral scanning method, comprising:
generating an energy ray beam toward an object to be scanned according to a preset ray generator;
performing conversion control on the voltage of the energy ray beam according to preset time intervals to obtain a high-voltage energy ray beam and a low-voltage energy ray beam;
using the high-voltage energy ray beam and the low-voltage energy ray beam to perform alternating interval irradiation according to a preset motion trajectory, and collecting attenuation signal data of the high-voltage energy ray beam and the low-voltage energy ray beam in real time; and
performing material decomposition on the attenuation signal data to obtain scanning imaging data of the object to be scanned.

2. The alternating dual-energy oral scanning method according to claim 1, wherein performing conversion control on the voltage of the energy ray beam according to preset time intervals to obtain a high-voltage energy ray beam and a low-voltage energy ray beam comprises:
calculating high-voltage irradiation time and low-voltage irradiation time according to a preset high voltage value, a preset low voltage value and preset fluctuation compensation constants;
performing output conversion control on the preset ray generator according to the preset high voltage value and the high-voltage irradiation time to obtain a high-voltage energy ray beam; and
performing output conversion control on the preset ray generator according to the preset low voltage value and the low-voltage irradiation time to obtain a low-voltage energy ray beam.

3. The alternating dual-energy oral scanning method according to claim 1, wherein using the high-voltage energy ray beam and the low-voltage energy ray beam to perform alternating interval irradiation according to a preset motion trajectory comprises:
calculating ray beam interval time according to a high voltage value of the high-voltage energy ray beam and a low voltage value of the low-voltage energy ray beam; and
performing alternating interval irradiation on the high-voltage energy ray beam and the low-voltage energy ray beam according to a preset motion trajectory according to the ray beam interval time.

4. The alternating dual-energy oral scanning method according to claim 1, wherein performing material decomposition on the attenuation signal data comprises:
converting the attenuation signal data of the high-voltage energy ray beam and the low-voltage energy ray beam into three-dimensional attenuation image data by back-projection operation to obtain high-voltage volume attenuation image data and low-voltage volume attenuation image data;
performing data decomposition on the high-voltage volume attenuation image data and the low-voltage volume attenuation image data according to preset base material water and preset base material bones to obtain decomposition coefficient image data of water and decomposition coefficient image data of bones; and
calculating virtual monoenergetic image data at a preset energy level according to decomposition coefficient images, and taking the virtual monoenergetic image data as scanning imaging data of the object to be scanned.

5. The alternating dual-energy oral scanning method according to claim 4, wherein performing data decomposition on the high-voltage volume attenuation image data and the low-voltage volume attenuation image data according to preset base material water and preset base material bones to obtain decomposition coefficient image data of water and decomposition coefficient image data of bones comprises:
decomposing the high-voltage volume attenuation image data into addition of a product of an attenuation coefficient of water under high-voltage conditions and the decomposition coefficient image data of water and a product of an attenuation coefficient of bones under high-voltage conditions and the decomposition coefficient image data of bones;
using the following formula to decompose the high-voltage volume attenuation image data into addition of the product of the attenuation coefficient of water under high-voltage conditions and the decomposition coefficient image data of water and the product of the attenuation coefficient of bones under high-voltage conditions and the decomposition coefficient image data of bones: $\left\{\begin{matrix}{μ}_{h} = {μ}_{ah} A + {μ}_{bh} B \\ {μ}_{l} = {μ}_{al} A + {μ}_{bl} B\end{matrix}\right.$
wherein *µₕ* and represent the high-voltage volume attenuation image data and the low-voltage volume attenuation image data respectively, *µ*ₐₕ and *µ*_{bh} represent the attenuation coefficient of water under high-voltage conditions and the attenuation coefficient of bones under high-voltage conditions respectively, *µ*ₐₗ and *µ*_{bl} represent the attenuation coefficient of water under low-voltage conditions and the attenuation coefficient of bones under low-voltage conditions respectively, and A and B represent the decomposition coefficient image data of water and the decomposition coefficient image data of bones respectively;
decomposing the low-voltage volume attenuation image data into addition of a product of an attenuation coefficient of water under low-voltage conditions and the decomposition coefficient image data of water and a product of an attenuation coefficient of bones under low-voltage conditions and the decomposition coefficient image data of bones;
calculating the decomposition coefficient image data of water and the decomposition coefficient image data of bones respectively according to the attenuation coefficient of water under high-voltage conditions, the attenuation coefficient of bones under high-voltage conditions, the attenuation coefficient of water under low-voltage conditions, the attenuation coefficient of bones under low-voltage conditions, the high-voltage volume attenuation image data and the low-voltage volume attenuation image data; and
using the following formula to calculate the decomposition coefficient image data of water and the decomposition coefficient image data of bones: $\left\{\begin{matrix}A = \frac{{μ}_{h} {μ}_{bl} - {μ}_{l} {μ}_{bh}}{{μ}_{ah} {μ}_{bl} - {μ}_{al} {μ}_{bh}} \\ B = \frac{{μ}_{h} {μ}_{al} - {μ}_{l} {μ}_{ah}}{{μ}_{bh} {μ}_{al} - {μ}_{bl} {μ}_{ah}}\end{matrix}\right.$
wherein A and B represent the decomposition coefficient image data of water and the decomposition coefficient image data of bones respectively, *µₕ* and represent the high-voltage volume attenuation image data and the low-voltage volume attenuation image data respectively, *µ*ₐₕ and *µ*_{bh} represent the attenuation coefficient of water under high-voltage conditions and the attenuation coefficient of bones under high-voltage conditions respectively, *µ*ₐₗ and *µ*_{bl} represent the attenuation coefficient of water under low-voltage conditions and the attenuation coefficient of bones under low-voltage conditions, respectively.

6. The alternating dual-energy oral scanning method according to claim 4, wherein calculating virtual monoenergetic image data at a preset energy level according to decomposition coefficient images comprises:
measuring an attenuation coefficient of water at a preset energy level and an attenuation coefficient of bones at a preset energy level respectively; and
calculating virtual monoenergetic image data at the preset energy level according to addition of a product of the attenuation coefficient of water at the preset energy level and the decomposition coefficient image data of water and a product of the attenuation coefficient of bones at the preset energy level and the decomposition coefficient image data of bones.

7. An alternating dual-energy oral scanning device, comprising:
a ray generation module configured to generate an energy ray beam toward an object to be scanned according to a preset ray generator;
a ray conversion module configured to perform conversion control on the voltage of the energy ray beam according to preset time intervals to obtain a high-voltage energy ray beam and a low-voltage energy ray beam;
an irradiation collection module configured to use the high-voltage energy ray beam and the low-voltage energy ray beam to perform alternating interval irradiation according to a preset motion trajectory, and collecting attenuation signal data of the high-voltage energy ray beam and the low-voltage energy ray beam in real time; and
a data decomposition module configured to perform material decomposition on the attenuation signal data to obtain scanning imaging data of the object to be scanned.

8. Computer equipment, comprising a memory, a processor and a computer program that is stored in the memory, wherein the processor executes the computer program to implement the steps of the alternating dual-energy oral scanning method according to any one of claims 1 to 6.

9. A computer-readable storage medium, storing a computer program thereon, wherein when being executed by a processor, the computer program implements the steps of the alternating dual-energy oral scanning method according to any one of claims 1 to 6.

10. A computer program product, comprising a computer program, wherein when being executed by a processor, the computer program implements the steps of the alternating dual-energy oral scanning method according to any one of claims 1 to 6.
